# EUROPEAN PATENT SPECIFICATION

(11) **EP 4 351 471 B1**
(45) Date of publication and mention of the grant of the patent: **21.05.2025**
(21) Application number: 22740535.4
(22) Date of filing: 08.06.2022
(51) Int. Cl.: A61C 7/36, A61F 5/56

(54) **MANDIBULAR ADVANCEMENT DEVICE AND SPACER KIT FOR A MANDIBULAR ADVANCEMENT DEVICE**
MANDIBULÄRE VORSCHUBVORRICHTUNG UND SPACER-KIT FÜR EINE MANDIBULÄRE VORSCHUBVORRICHTUNG
DISPOSITIF D'AVANCEMENT MANDIBULAIRE ET KIT D'ESPACEMENT POUR UN DISPOSITIF D'AVANCEMENT MANDIBULAIRE

(30) Priority: 11.06.2021 IT 202100015263
(43) Date of publication of application: 17.04.2024
(73) Proprietor: Teor Srl, 47923 Rimini (RN) (IT); Anelli, Fabrizio, 47900 Rimini (RN) (IT)
(72) Inventor: ANELLI, Fabrizio, 47900 Rimini (RN) (IT)
(74) Representative: Garavelli, Paolo
(86) International application number: PCT/IT2022/050166
(87) International publication number: WO 2022/259283

(56) References cited:
- US-A1- 2011 168 187
- US-A1- 2012 227 750
- US-A1- 2013 112 210
- US-A1- 2016 100 915

## Description

### Technical Field

The present invention relates to a mandibular advancement device.

Furthermore, the present invention relates to a spacer kit for a mandibular advancement device.

In general, the present invention relates to intraoral devices, stops or mouthpiece po-sitioners, for example for both the lower and upper arches, devices acting between the upper and lower teeth.

### Background Art

Patent application EP 3 383 328 A4 relates to an oral apparatus for treating snoring and/or night apnea in a subject, comprising an upper tray and a lower tray, the upper tray provided with a right and left side insert, the rear end of each insert reversibly attached to the front end of a respective lateral projection through an interference fit, the rearward projection may be a wedge having sides extending laterally and rearwardly in which case the recess is wedge-shaped and configured to receive and retain the projection in a male-female manner, a plurality of pairs of inserts, with at least some of the pairs of inserts having side sides that are of different length than the side sides of other pairs of inserts to allow customization of the positioning of the upper jaw of a user with respect to the lower jaw of the user during use of the present apparatus, the lower tray such as the upper tray includes a receptacle and a pair of upwardly extending projections contacting the front ends of the respective inserts, the rear side of the projection contacts the front surface of the insert to limit, stop, the anterior movement of the jaw and the corresponding posterior movement of the jaw, thus maintaining the maxilla and mandibula in an orientation favorable to the prevention or improvement of the nocturnal apnea, the use of laterally positioned axial inserts are configured to attach to each side side of the upper tray and to engage the rear side of the upward projection of the lower tray on an engagement surface of the insert, each insert comprises an engaging surface and a rear attachment end opposite the front engagement surface, the insert further comprises an inner surface which comes into contact or is adjacent to a respective lateral outer surface of the upper tray and an outer surface opposite the outer surface. the forward facing engagement surface of the side projection is designed to cooperate with the engagement surface of the upper tray insert so as to mechanically retain the insert on the upper tray. the rear attachment end and the front connection end are preferably provided with locking members which fit together to hold the insert on the upper tray, the attachment end of the insert engages with the connection end of the upper tray in a male-female manner. in such a way that a laterally flared wedge fits into a cavity formed at the connecting end, the insert could alternatively be provided with a groove and the connecting end could be configured with a forward projecting tab. or other configurations may be provided to mechanically attach the insert to the upper tray, the sides of the wedge preferably form an interference fit with the groove or recess in order to firmly retain the insert in the present apparatus, the insert is formed of an elastomeric material, and the tongue of the attachment end is formed with slightly larger dimensions than the recess, so that when the tab is pushed into the recess, it exerts a force which helps to hold it inside the recess, once it is fixed to the upper tray, the forward facing engagement surface of the insert is positioned to contact the rearward facing rear side of the projection when the present apparatus is worn by a user.

In order to be able to adjust the relative position of a user's jaw and jaw when the present apparatus is worn, inserts of different lengths, formed with sides of different length, may be used. such that the engaging surfaces of different inserts may extend further forward or rearward, depending on the user's needs, the inserts labeled 1 to 6 comprise increasing length sides, thereby, The jaw of a user may be positioned relatively anteriorly by the use of higher number inserts, i.e. with longer lengths, or may be positioned relatively posteriorly by the use of lower number inserts with shorter sides.

Patent KR 10 1590813 B1 relates to an orthodontic appliance comprising a tooth guard, an engaging portion provided in an end portion of the tooth guard and a regulator for correcting the jaw joint, The upper engaging portion forms a lateral projection and supports the posterior surface of the regulator to the anterior surface of the projection, a tapered surface acts as a support surface for supporting the posterior surface of the regulator coupled to the posterior side of the lower jaw of the mandible shield, a lash adjuster includes a plurality of lash adjusters having widths different from those of the adjuster so that the lash adjuster having a width adequate according to the calibration state of the user and calibrator can be used, this regulator prevents the closure of the airways of the upper portion of the rear tongue or pharyngeal and of the rear pharyngeal according to the correction state of the calibrator jaw or during sleep, and the articulation is maintained in a state in which it exits in all directions, the jaw correction device using the adjustment device can be selectively applied to a patient not to snoring only during sleep, or to a patient requiring correction of the maxillary joint itself even if snoring is not serious, moreover, it is possible to selectively change the position of the jaw joint based on the calibration state and the progress of the correction, the regulator can adjust the selected regulator among the plurality of regulators whose widths from D 1 to D N are varied according to the calibration state of the subject, So that the position of the jaw can be shifted in all directions, the regulators are composed of a plurality of regulators of the same structure, but the lengths from D 1 to D N of the bodies are different from each other. Widths from D 1 to D N vary in width from D 1 <D 2 <D 3 to D N.

EP 3 232 980 B1 relates to an oral apparatus provided with axial inserts configured to attach to a tray and to engage a pad on a further tray of the present apparatus, the inserts attached to the upper tray engage the lower bite pad of the lower tray. the pads and inserts cooperate together to limit and define the relative antero-posterior positions of the jaw and jaw of a user of the present apparatus, each insert comprises an engagement surface, a right side, a left side and an attachment end opposite the engagement surface, the insert further comprises a coronal surface and a contact surface of the tray opposite the coronal surface.

The attachment end of the insert engages with the connection end of the upper in a male-female manner, so that a laterally flared wedge fits into a cavity formed in the connection end, the insert could alternatively be provided with a groove and the connecting end could be configured with a forward projecting tab, or a differently configured projection could be used, such as a rounded ball adapted to fit into a corresponding rounded hole. Other configurations may also be used to mechanically attach the insert to the upper tray, in an alternative, an insert could be attached to the upper bite pad with an expansion screw, thereby allowing the length of the insert to be further adjusted, as desired. the insert could alternatively be attached to the lower tray, such as the lower bite pad, the sides of the wedge preferably form an interference fit with the groove or recess in order to hold the insert firmly in the present apparatus, in a preferred embodiment, the insert and/or the upper pad are formed of an elastomeric material, and the tongue of the attachment end is formed with slightly larger dimensions than the cavity, to be able to adjust the relative position of a user's jaw and jaw when the present apparatus is worn, inserts of different lengths may be used, the inserts may be formed with sides of different lengths, so that the engagement surfaces of different inserts extend further forward or backward, depending on the needs of the user.

The jaw of a user may be positioned relatively anteriorly by the use of longer inserts, i.e. with longer side sides, or may be positioned relatively posteriorly by the use of shorter side inserts. A series of inserts may be provided for use with the present apparatus having side sides differing in 1 mm increments, 0.5 mm increments or 0.25 mm increments, for example.

US2016/100915 discloses a mandibular repositioning device including upper and lower assemblies, each with adjustable wedges secured by set screws in buccal tubes connected to molar brackets, enabling controlled repositioning of the mandibular jaw through interlocking rounded contact surfaces.

### Summary of Invention

### Technical Problem

The insert known from the prior art has a flared wedge laterally inserted into a cavity formed at the connecting end, the insert could alternatively be provided with a groove and the connecting end could be configured with a forward projecting tab. or other configurations may be provided to mechanically attach the insert to the upper tray, the sides of the wedge preferably form an interference fit with the groove or recess in order to firmly retain the insert in the present apparatus. However, this solution involves a concentrated overmold in the groove which reduces the possibility of choosing the insert material.

### Solution to Problem

The aim of the present invention is to solve the above problems of the prior art by providing a solution compatible with the material with which the insert is to be made.

The above and other objects and advantages of the invention, as will appear from the following description, are achieved with a dental device such as that described in claim 1 and with a kit of pairs of spacers such as that described in claim 8. Preferred embodiments and non-trivial variations of the present invention are the subject of the dependent claims.

It is understood that all the appended claims form an integral part of the present description. It will be immediately obvious that countless variations and modifications (for example, relating to shape, dimensions, can be made to what has been described herein. arrangements and parts with equivalent functionalities) without departing from the scope of the invention as set forth in the appended claims.

### Brief Description of Drawings

The present invention will be better described by some preferred embodiments, given by way of non-limiting example, with reference to the attached drawings.
[Fig.1] shows an isometric view of an embodiment of the dental device according to the present invention.
[Fig. 2) shows a side view of the previous figure.
[Fig. 3) shows a plan view of an upper part of an embodiment of the dental device according to the present invention.
[Fig.4]) shows a plan view of a lower part of an embodiment of the dental device according to the present invention.
[Fig.5] shows an isometric view of a first component of an embodiment of the dental device according to the present invention.

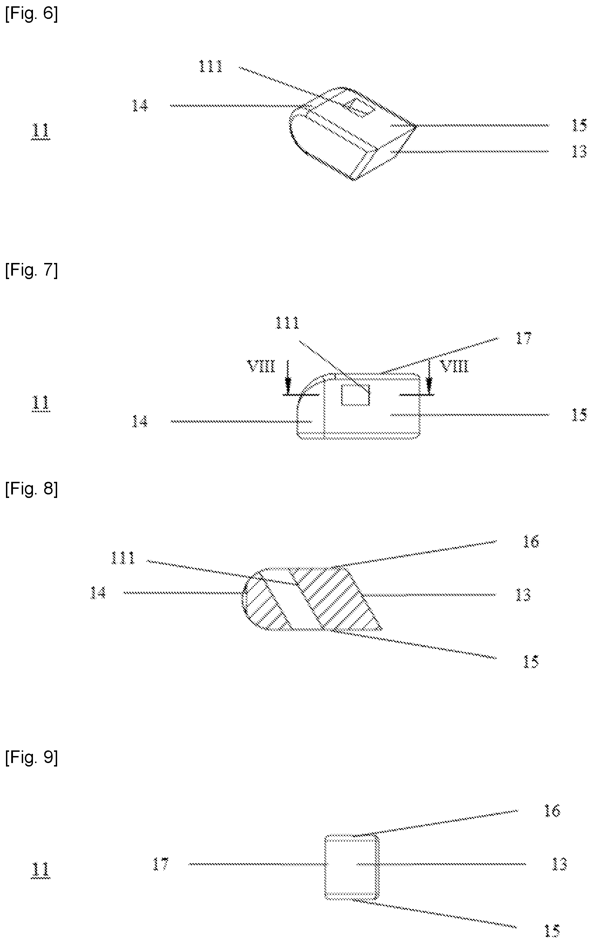

[Fig.6] shows an isometric view of a component specular to that of the previous figure.
[Fig.7] shows a plan view of the previous figure.
[Fig.8] shows a sectional view according to the line VIII-VIII of the previous figure.
[Fig.9] shows a front view of [Fig.6].

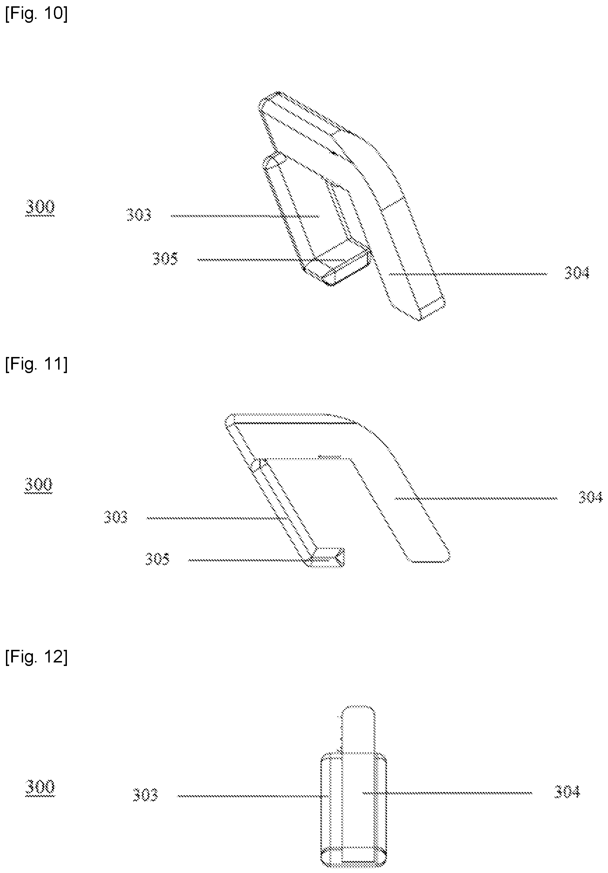

[Fig.10] shows an isometric view of a second component of an embodiment of the dental device according to the present invention.
[Fig.11] shows a side view of the previous figure.
[Fig.12] shows a front view of [Fig.10].

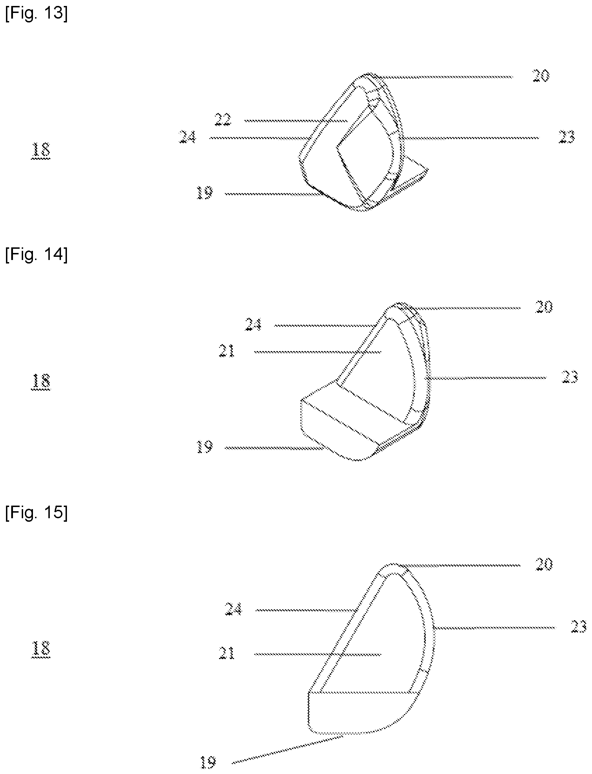

[Fig.13] shows an isometric view of a third component of an embodiment of the dental device according to the present invention.
[Fig.14] shows an isometric view of a component specular to that of the previous figure.
[Fig. 15) shows a side view of the previous figure.

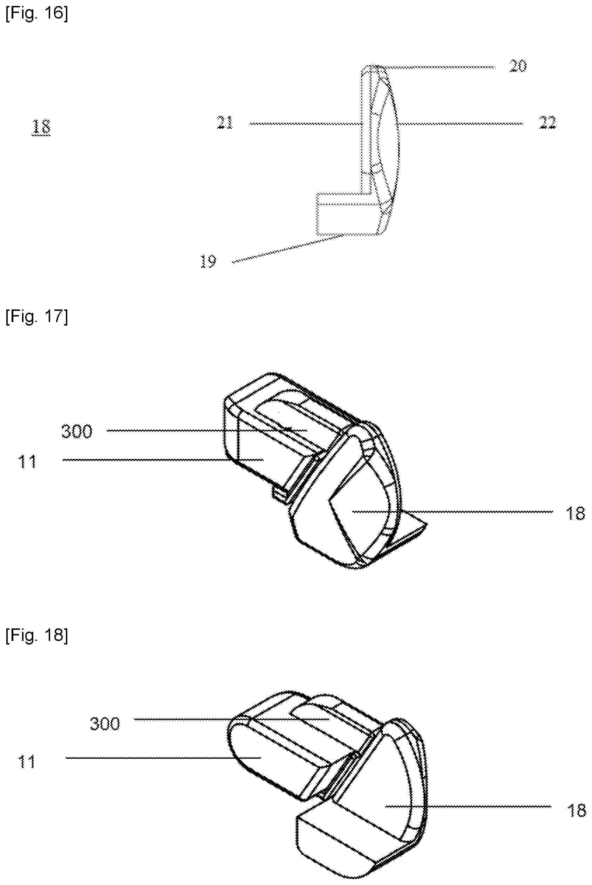

[Fig.16] shows a front view of [Fig.14].
[Fig.17] shows an isometric view of a relevant portion of an embodiment of the dental device according to the present invention.
[Fig.18] shows an isometric view of a portion specular to that of the previous figure.
[Fig.19] shows an exploded isometric view of the preceding figure.
[Fig. 20) shows an isometric view of an embodiment of a part of the spacer pair kit according to the present invention.
[Fig.21] shows an isometric view of an embodiment of a mirror part of the spacer pair kit according to the present invention.

### Description of Embodiments

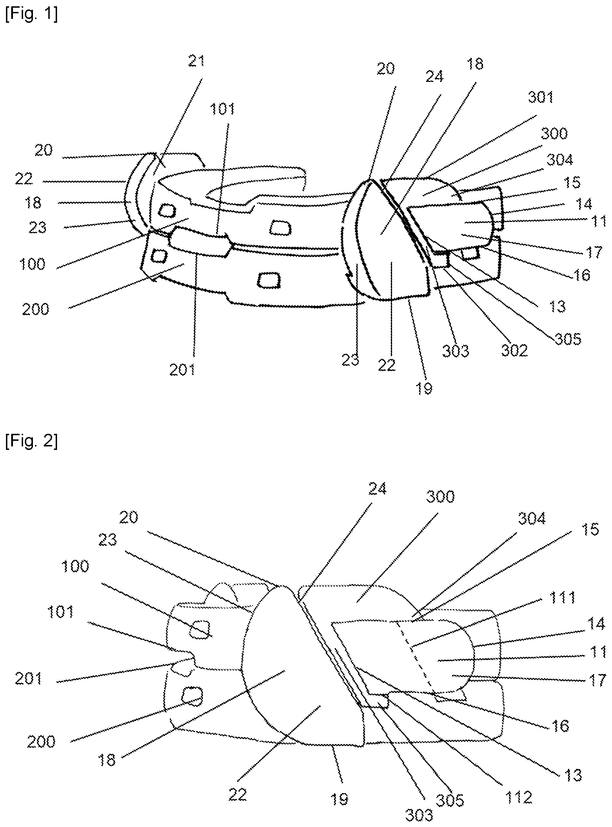

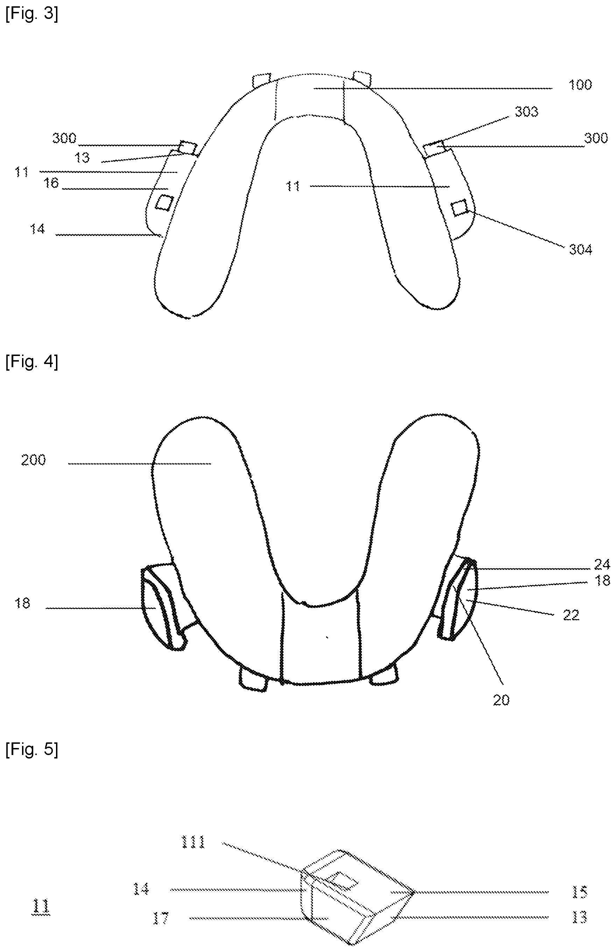

With reference to [Fig.1], [Fig.2], [Fig.3], [[Fig.4]), it can be noted that a dental device comprises an upper tray 100 and a lower tray (bíte) 200. The dental device is formed in a front portion, a rear portion, a right side, a left side, a buccal side, a lingual side, an upper and lower coronal surface, an upper and lower inner surface, an upper and lower outer surface, a plurality of upper and lower receptacles, each receptacle delimited by an inner surface, an upper incisor surface 101, a lower incisor surface 201.

The upper tray 100 comprises:
a pair of right and left inserts 11, each insert 11 is connected to the buccal side of the right and left side of the upper tray 100 in a rear portion of the upper tray 100. each insert 11 is delimited by a front end with an engagement surface 13, a rear end 14, an upper surface 15, a lower surface 16 and a buccal surface 17;
a pair of right and left spacers 300, each spacer 300 including a front end 303 and a rear end 304, each spacer 300 is configured to be reversibly secured to the respective insert 11.

The lower tray 200 comprises:
a pair of right and left mandibular cushions 18, each upwardly projecting mandibular cushion 18 comprising a proximal end 19, a distal end 20, an inner surface 21, an outer surface 22, a front side 23 and a rear side 24, each jaw cushion 18 connected to the proximal end 19 to the right and left side of the lower tray 200, the rear side 24 faces the front surface of the front end 303 of the respective spacer 300 of the right and left side of the upper tray 100.

The dental device allows limiting the forward positioning of the upper tray 100 relative to the lower tray 200 by contacting the front surface of the front end 303 of the pair of right and left spacers 300 with the rear side surface 24 of the pair of right hand bolar cushions and left 18.

At least a portion of the upper incisor surface 101 and the lower incisor surface 201 is not in contact when the upper coronal surface contacts the lower coronal surface in a rear portion of the apparatus. This allows to form a front opening allowing air flow through the front opening during use of the device.

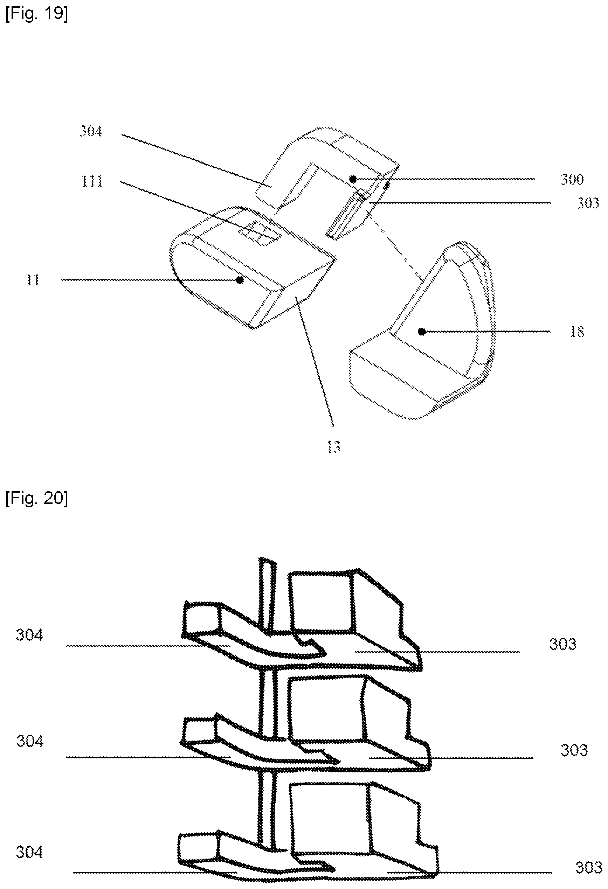

Advantageously, with reference to Figures [Fig.5] to [Fig.19], each insert 11 comprises at least one grooved profile 111 parallel to the front end with an engagement surface 13, each insert 11 adapted to receive the respective spacer 300 by means of the front end 303 inserted along the engagement surface 13 and the rear end 304 inserted along the grooved profile 111.

Each spacer 300 is shaped as an elastically deformable clip, the elastically deformable clip is inserted against the respective insert 11.

Such at least one grooved profile 111 occupies a cavity which passes through the insert 11 from the upper surface 15 to the lower surface 16.

Alternatively, the grooved profile 111 occupies the rear end 14 of the insert 11, not shown.

A variant is that according to which each spacer 300 is snap-locked with respect to the respective insert 11 by means of a projection 305 of the front end 303 lying in a suitable seat 112 of the respective insert 11, the front end 303 elastically deforming while the projection 305 slides along the grooved profile 111.

A series of pairs of spacers such as the pair of right and left spacers 300 are mutually interchangeable right spacers and mutually interchangeable left spacers, the right and left spacers being distinguished by the thickness of the front end 303 with the same front surface as the right and left spacer 300 contacting the surface of the rear side 24 of the respective mandibular cushion 18, thus limiting the forward positioning of the upper tray 100 relative to the lower tray 200.

The device derived from a digital model obtained from an optical impression is obtained from additive manufacturing of three-dimensional printing of a polymer, the upper tray 100 and lower tray 200 precisely reproducing the negative of the teeth cusps stabilized at the pressure arches in order to be able to maintain their position without resorting to auxiliary retention components.

A use of the dental device allows to relieve the night apnea of the subject using the device by forming a front opening allowing air flow through the front opening during use of the device.

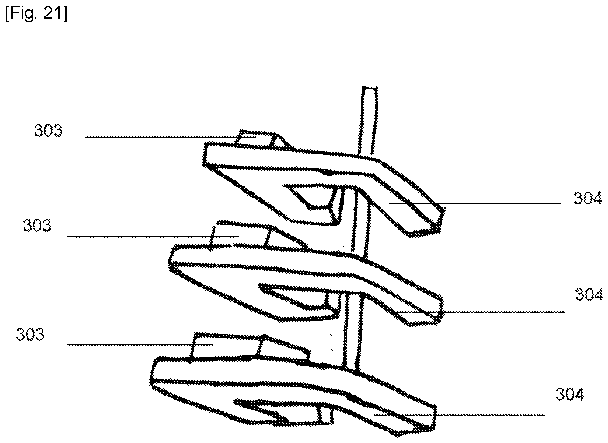

Referring to [Fig.20], [Fig.21], a set of spacer pairs for a dental device is a package of a set of spacer pairs.

### Examples

Mandibular advancement device in cases of night apnea.

This is an upper bite and a lower bite applied to the dental arches after the dental impression.

To these two bits a feeding mechanism is applied with inserts thickened by various mm, the inserts are interchangeable in order to be able to propel the jaw.

The dentist can change them according to the millimeter calibration.

The anterior wing mechanism located in the lower bite intersects with the upper postero mechanism through a 70 degree inclined plane which allows the patient to advance the jaw without any difficulty.

The device is completely designed in 3D and manufactured from biocompatible polyamide.

## Claims

1. Dental device, comprising an upper bite plane (100) and a lower bite plane (200), shaped in an anterior portion, a posterior portion, a right side, a left side, a buccal side, a lingual side, a upper and lower coronal surface, an upper and lower inner surface, an upper and lower outer surface, a plurality of upper and lower receptacles, each receptacle delimited by an inner surface, an upper incisal surface (101), a lower incisal surface (201),
the upper bite plane (100) comprising
- a pair of right and left inserts (11), each insert (11) connected to the buccal side of the right and left side of the upper bite plane (100) in a rear portion of the upper bite plane (100), each insert (11) delimited by a front end with an engagement surface (13), a rear end (14), an upper surface (15), a lower surface (16) and a buccal surface (17),
- a pair of right and left spacers (300), each spacer (300) comprising a front end (303) and a rear end (304), each spacer (300) configured to be reversibly fixed to the respective insert ( 11),
the lower bite plane (200) comprising
- a pair of right and left mandibular cushions (18), each mandibular cushion (18) projecting upwards, comprising a proximal end (19), a distal end (20), an internal surface (21), an external surface (22), an anterior side (23) and a posterior side (24), each mandibular cushion (18) connected at the proximal end (19) to the right and left side of the lower bite plane (200), the posterior side (24 ) faces the front surface of the front end (303) of the respective spacer (300) of the right and left side of the upper bite plane (100),
the dental device suitable for limiting the forward positioning of the upper bite plane (100) with respect to the lower bite plane (200) by means of
- the contact of the anterior surface of the anterior end (303) of the pair of right and left spacers (300) with the surface of the posterior side (24) of the pair of right and left mandibular cushions (18),
- at least a portion of the upper incisal surface (101) and the lower incisal surface (201) not being in contact when the upper coronal surface comes into contact with the lower coronal surface in a posterior portion of the appliance, thus forming an anterior opening allowing airflow through the front opening when using the device,
the dental device **characterized in that** each insert (11) comprises at least one grooved profile (111) parallel to the anterior end with an engagement surface (13), each insert (11) adapted to receive the respective spacer (300) through the front end (303) inserted along the engagement surface (13) and the rear end (304) inserted along said grooved profile (111).

2. Device according to the previous claim, **characterized in that** each spacer (300) is shaped like an elastically deformable clip, the elastically deformable clip being inserted close to the respective insert (11).

3. Device according to one of the previous claims, **characterized in that** said at least one grooved profile (111) occupies a cavity that crosses the insert (11) from the upper surface (15) to the lower surface (16).

4. Device according to claim 1, 2, **characterized in that** said at least one grooved profile (111) occupies the rear end (14) of the insert (11).

5. Device according to one of the previous claims, **characterized in that** each spacer (300) is snap-locked with respect to the respective insert (11) by means of a projection (305) of the front end (303) lying in a suitable seat (112) of the respective insert (11), the front end (303) elastically deformed while said projection (305) slides along the grooved profile (111).

6. Device according to one of the previous claims, **characterized in that** it comprises a series of pairs of spacers such as the pair of right and left spacers (300), the right spacers being reciprocally interchangeable, the left spacers being reciprocally interchangeable, the right and left spacers distinguished by the thickness of the anterior end (303) with the same anterior surface of the spacer (300) right and left which contacts the surface of the posterior side (24) of the respective mandibular cushion (18), thus limiting the forward positioning of the upper bite plane (100) than the lower bite plane (200).

7. Device according to one of the previous claims, **characterized in that** it is obtained from the additional manufacturing of three-dimensional printing of a polymer, the upper (100) and lower (200) bite plane reproducing precisely the negative of the dental cuspides stabilized on the pressure arches to be able to maintain themselves in position without resorting to auxiliary retention components.

8. Kit of pairs of spacers for a dental device according to claim 6, **characterized in that** it consists of a package of a series of pairs of spacers according to claim 6.

## Patentansprüche

1. Dentalgerät, bestehend aus einem oberen Tablett (100) und einem unteren Tablett (200), geformt in einen vorderen Abschnitt, einen hinteren Abschnitt, eine rechte Seite, eine linke Seite, eine bukkale Seite, eine linguale Seite, eine obere koronale Oberfläche und unten, eine obere und untere Innenfläche, eine obere und untere Außenfläche, mehrere obere und untere Behälter, wobei jeder Behälter durch eine Innenfläche begrenzt ist, eine obere Schneidefläche (101), eine untere Schneidefläche (201),
wobei die obere Ablage (100) Folgendes umfasst:
- ein Paar rechter und linker Einsätze (11), wobei jeder Einsatz (11) mit der bukkalen Seite der rechten und linken Seite des oberen Löffels (100) in einem hinteren Abschnitt des oberen Löffels (100) verbunden ist, wobei jeder Einsatz (11) begrenzt durch ein vorderes Ende mit einer Eingriffsfläche (13), ein hinteres Ende (14), eine obere Fläche (15), eine untere Fläche (16) und eine bukkale Fläche (17),
- ein Paar rechter und linker Abstandshalter (300), wobei jeder Abstandshalter (300) ein vorderes Ende (303) und ein hinteres Ende (304) umfasst, wobei jeder Abstandshalter (300) so konfiguriert ist, dass er reversibel an dem jeweiligen Einsatz (11) befestigt werden kann,
wobei die untere Ablage (200) Folgendes umfasst:
- ein Paar rechter und linker Unterkieferpolster (18), wobei jedes Unterkieferpolster (18) nach oben ragt und ein proximales Ende (19), ein distales Ende (20), eine Innenfläche (21) und eine Außenfläche (22) umfasst. , eine vordere Seite (23) und eine hintere Seite (24), wobei jedes Unterkieferkissen (18) am proximalen Ende (19) mit der rechten und linken Seite des unteren Tabletts (200) verbunden ist, die Rückseite (24) ist der Vorderfläche des vorderen Endes (303) des jeweiligen Abstandshalters (300) der rechten und linken Seite des oberen Tabletts (100) zugewandt,
wobei das zahnmedizinische Gerät dazu ausgelegt ist, die Vorwärtspositionierung des oberen Tabletts (100) in Bezug auf das untere Tablett (200) zu begrenzen
- der Kontakt der Vorderfläche des vorderen Endes (303) des Paars aus rechten und linken Abstandshaltern (300) mit der Oberfläche der Rückseite (24) des Paares aus rechten und linken Unterkieferpolstern (18),
- mindestens ein Teil der oberen Schneidefläche (101) und der unteren Schneidefläche (201) stehen nicht in Kontakt, wenn die obere koronale Fläche die untere koronale Fläche in einem hinteren Teil der Vorrichtung berührt, wodurch eine vordere Öffnung gebildet wird, die einen Luftstrom ermöglicht durch die vordere Öffnung, während Sie das Gerät verwenden,
wobei die zahnmedizinische Vorrichtung **dadurch gekennzeichnet ist, dass** jeder Einsatz (11) mindestens ein Rillenprofil (111) parallel zum vorderen Ende mit einer Eingriffsfläche (13) aufweist, wobei jeder Einsatz (11) in der Lage ist, den jeweiligen Abstandshalter (300) über die aufzunehmen vorderes Ende (303) mit Gewinde entlang der Eingriffsfläche (13) und hinteres Ende (304) mit Gewinde entlang des Rillenprofils (111).

2. Dentalgerät nach dem vorherigen Anspruch, **dadurch gekennzeichnet, dass** jeder Abstandshalter (300) die Form einer elastisch verformbaren Klammer hat, wobei die elastisch verformbare Klammer nahe am jeweiligen Einsatz (11) eingesetzt ist.

3. Dentalgerät nach einem der vorhergehenden Ansprüche, **dadurch gekennzeichnet, dass** das mindestens eine Rillenprofil (111) einen Hohlraum einnimmt, der durch den Einsatz (11) von der oberen Fläche (15) zur unteren Fläche (16) verläuft.

4. Dentalgerät nach den Ansprüchen 1, 2, **dadurch gekennzeichnet, dass** das mindestens eine Rillenprofil (111) das hintere Ende (14) des Einsatzes (11) einnimmt.

5. Dentalgerät nach einem der vorhergehenden Ansprüche, **dadurch gekennzeichnet, dass** jeder Abstandshalter (300) über einen Vorsprung (305) des vorderen Endes (303), der in einer speziellen Aufnahme (112) des jeweiligen Einsatzes (11) liegt, wobei das vordere Ende (303) elastisch verformt wird, während der Vorsprung (305) entlang des Rillenprofils (111) gleitet.

6. Dentalgerät nach einem der vorhergehenden Ansprüche, **dadurch gekennzeichnet, dass** sie eine Reihe von Abstandshalterpaaren wie das Paar rechter und linker Abstandshalter (300) umfasst, wobei die rechten Abstandshalter untereinander austauschbar sind, die linken Abstandshalter untereinander austauschbar sind Abstandshalter rechts und links unterscheiden sich durch die Dicke des vorderen Endes (303), wobei die gleiche Vorderfläche des rechten und linken Abstandshalters (300) die Oberfläche der Rückseite (24) des jeweiligen Kissens berührt Unterkiefer (18), wodurch die Vorwärtspositionierung des oberen Löffels (100) in Bezug auf den unteren Löffel (200) begrenzt wird.

7. Dentalgerät nach einem der vorhergehenden Ansprüche, **dadurch gekennzeichnet, dass** sie durch additive Fertigung mit dreidimensionalem Druck eines Polymers hergestellt wird, wobei die obere (100) und untere (200) Schale das Negativ der stabilisierten Zahnhöcker genau reproduzieren durch Druck auf die Bögen ausgeübt werden, um die Position halten zu können, ohne auf zusätzliche Retentionskomponenten zurückgreifen zu müssen.

8. Kit aus Abstandshalterpaaren für ein Dentalgerät nach Anspruch 6, **dadurch gekennzeichnet, dass** er aus einer Packung einer Reihe von Abstandshalterpaaren nach Anspruch 6 besteht.

## Revendications

1. Dispositif dentaire, comprenant un plateau supérieur (100) et un plateau inférieur (200), conformés en une partie antérieure, une partie postérieure, un côté droit, un côté gauche, un côté buccal, un côté lingual, une face coronale supérieure. et inférieure, une surface interne supérieure et inférieure, une surface externe supérieure et inférieure, une pluralité de réceptacles supérieur et inférieur, chaque réceptacle étant délimité par une surface interne, une surface incisive supérieure (101), une surface incisive inférieure (201),
le plateau supérieur (100) comprenant
- une paire d'inserts droit et gauche (11), chaque insert (11) relié au côté buccal du côté droit et gauche du plateau supérieur (100) dans une partie arrière du plateau supérieur (100), chaque insert (11) délimité par une extrémité avant avec une surface d'engagement (13), une extrémité arrière (14), une surface supérieure (15), une surface inférieure (16) et une surface buccale (17),
- une paire d'entretoises droite et gauche (300), chaque entretoise (300) comprenant une extrémité avant (303) et une extrémité arrière (304), chaque entretoise (300) configurée pour être fixée de manière réversible à l'insert respectif (11),
le plateau inférieur (200) comprenant
- une paire de coussinets mandibulaires droit et gauche (18), chaque coussinet mandibulaire (18) faisant saillie vers le haut, comprenant une extrémité proximale (19), une extrémité distale (20), une surface interne (21), une surface externe (22) , un côté antérieur (23) et un côté postérieur (24), chaque coussinet mandibulaire (18) étant relié par l'extrémité proximale (19) aux côtés droit et gauche du plateau inférieur (200), le côté arrière (24) fait face à la surface avant de l'extrémité avant (303) de l'entretoise respective (300) des côtés droit et gauche du plateau supérieur (100),
le dispositif dentaire conçu pour permettre de limiter le positionnement vers l'avant du plateau supérieur (100) par rapport au plateau inférieur (200) par
- le contact de la surface avant de l'extrémité antérieure (303) de la paire d'entretoises droite et gauche (300) avec la surface du côté arrière (24) de la paire de coussins mandibulaires droit et gauche (18),
- au moins une partie de la surface incisive supérieure (101) et de la surface incisive inférieure (201) n'étant pas en contact lorsque la surface coronale supérieure entre en contact avec la surface coronale inférieure dans une partie postérieure de l'appareil, formant ainsi une ouverture antérieure permettant la circulation de l'air par l'ouverture avant lors de l'utilisation de l'appareil,
le dispositif dentaire **caractérisé en ce que** chaque insert (11) comprend au moins un profil rainuré (111) parallèle à l'extrémité avant avec une surface d'engagement (13), chaque insert (11) pouvant recevoir l'entretoise respective (300) via le l'extrémité avant (303) est filetée le long de la surface d'engagement (13) et l'extrémité arrière (304) est filetée le long dudit profil rainuré (111).

2. Dispositif selon la revendication précédente, **caractérisé en ce que** chaque entretoise (300) est conformée comme une pince élastiquement déformable, la pince élastiquement déformable étant insérée à proximité de l'insert respectif (11).

3. Dispositif selon l'une des revendications précédentes, **caractérisé en ce que** ledit au moins un profilé rainuré (111) occupe une cavité qui traverse l'insert (11) depuis la surface supérieure (15) jusqu'à la surface inférieure (16).

4. Dispositif selon les revendications 1, 2, **caractérisé en ce que** ledit au moins un profilé rainuré (111) occupe l'extrémité arrière (14) de l'insert (11).

5. Dispositif selon l'une des revendications précédentes, **caractérisé en ce que** chaque entretoise (300) est encliquetée par rapport à l'insert respectif (11) via une saillie (305) de l'extrémité avant (303) reposant dans un logement spécial (112) de l'insert respectif (11), l'extrémité avant (303) se déforme élastiquement tandis que ladite saillie (305) coulisse le long du profil rainuré (111).

6. Dispositif selon l'une des revendications précédentes, **caractérisé en ce qu'**il comprend une série de paires d'entretoises telles que la paire d'entretoises droite et gauche (300), les entretoises droites étant mutuellement interchangeables, les entretoises gauches étant mutuellement interchangeables, les les entretoises droite et gauche se distinguent par l'épaisseur de l'extrémité avant (303), la même surface avant des entretoises droite et gauche (300) étant en contact avec la surface du côté arrière (24) du coussin respectif mandibulaire (18), limitant ainsi le positionnement vers l'avant du plateau supérieur (100) par rapport au plateau inférieur (200).

7. Dispositif selon l'une des revendications précédentes, **caractérisé par** le fait d'être obtenu à partir d'une fabrication additive d'impression tridimensionnelle d'un polymère, le plateau supérieur (100) et inférieur (200) reproduisant avec précision le négatif des cuspides dentaires stabilisé. sur les arceaux par pression pour pouvoir maintenir en position sans recourir à des éléments auxiliaires de rétention.

8. Kit de paires d'espaceurs pour dispositif dentaire selon la revendication 6, **caractérisé en ce qu'**il est constitué d'un emballage d'une série de paires d'espaceurs selon la revendication 6.
